# FASCICULE DE BREVET EUROPEEN

(11) **EP 1 313 453 B1**
(45) Date de publication et mention de la délivrance du brevet: **21.05.2008**
(21) Numéro de dépôt: 01984141.0
(22) Date de dépôt: 05.07.2001
(51) Int. Cl.: A61K 9/16, A61K 47/30, A61K 47/38, A61K 31/727

(54) **VECTEURS PARTICULAIRES DESTINES A AMELIORER L'ABSORPTION ORALE DE PRINCIPES ACTIFS**
TRÄGER IN PARTIKELFORM ZUR VERBESSERUNG DER ORAL- ABSORPTION VON WIKSTOFFEN
PARTICULATE VECTORS FOR IMPROVING ORAL ABSORPTION OF ACTIVE PRINCIPLES

(30) Priorité: 07.07.2000 FR 0008902
(43) Date de publication de la demande: 28.05.2003
(73) Titulaire: LABORATORIOS FARMACEUTICOS ROVI, S.A., 28037 Madrid (ES)
(72) Inventeur: Maincent, Philippe, 54000 Nancy (FR); Ubrich, Nathalie, 54000 Nancy (FR); Vigneron, Claude, 54000 Nancy (FR)
(74) Mandataire: Elzaburu Marquez, Alberto
(86) Numéro de dépôt international: PCT/FR2001/002159
(87) Numéro de publication internationale: WO 2002/003960

(56) Documents cités:
- WO-A-92/11844
- WO-A-94/14420
- WO-A-96/28143
- FR-A- 2 769 853

## Description

La présente invention concerne des vecteurs particulaires destinés à augmenter l'absorption des principes actifs après administration par voie orale chez l'homme ou l'animal.

Il est actuellement admis que la plupart des principes actifs qui seront utilisés dans les 30 prochaines années n'ont pas encore été découverts. De plus les études prospectives s'accordent pour penser que la majorité de ces futurs principes actifs, issus des techniques de génie biologique, seront des peptides et/ou des protéines. Ces nouvelles drogues seront extrêmement actives avec des doses efficaces qui seront de l'ordre du microgramme ou moins. Quelques drogues, principalement des peptides, sont déjà sur le marché nord américain ou européen (analogues de la LH/RH, hormone de croissance, streptokinase, anticorps...). Plus d'une centaine de peptides et de protéines sont actuellement en cours d'essais cliniques chez l'homme.

Les peptides et les protéines, actuellement sur le marché, présentent un certain nombre d'inconvénients qui limitent leur emploi chez l'homme :
- la seule voie d'administration est la voie parentérale (intraveineuse, sous cutanée, intramusculaire) et
- leur demi-vie d'élimination dans l'organisme est brève, ce qui exige des administrations multiples.

Il n'existe pas actuellement de formes pharmaceutiques permettant l'administration orale des protéines et des peptides alors que des formes multiparticulaires, destinées à la voie parentérale sont présentes sur le marché depuis plusieurs années (Enantone® des laboratoire Takeda, Sandostatine® des laboratoires Novartis).

L'absence de formes destinées à la voie orale s'explique par la sensibilité des peptides et protéines aux sucs digestifs qui dégradent les médicaments protéiques de la même façon que les protéines alimentaires; il en résulte une absence pratiquement totale d'absorption due à la destruction initiale. La voie orale étant la voie la plus commune et la plus facilement acceptée chez l'homme, la mise au point d'une forme permettant de protéger les peptides et les protéines de l'inactivation par les sucs digestifs tout en permettant l'absorption gastro-intestinale représenterait une avancée thérapeutique majeure en ce début de siècle.

C'est en raison de cette inactivation par les enzymes protéolytiques du tractus gastro-intestinal que l'insuline, un peptide de 51 acides aminés, est administrée depuis presque 80 ans par voie parentérale. Certains essais d'amélioration de l'absorption des peptides et des protéines par voie orale ont été effectués. Plusieurs travaux de la littérature citent par exemple une augmentation de l'absorption de l'insuline par voie orale quand cette dernière est incorporée dans des nanocapsules de poly(isobutylcyanoacrylate) (MICHEL C., et al., J. Pharm. Pharmacol., 43, (1991), 1-5 ; DAMGE C., et al. Diabètes, 37, (1988), 246-251). Ces nanocapsules ont une structure vésiculaire correspondant à des nanogouttelettes d'huile entourée par une très fine membrane du polymère. Cependant le polymère choisi est connu pour son caractère toxique au niveau cellulaire ce qui ne peut permettre d'envisager une administration répétée pendant plusieurs années. De même, la présence d'huile dans le coeur des nanocapsules poserait, au long terme, des problèmes de toxicité au niveau des sites d'administration qui ne permettent pas d'envisager la mise sur le marché de nanocapsules d'insuline. D'autre part, l'effet hypoglycémiant n'est observé qu'au bout de 2 jours probablement dû à un franchissement très limité des particules à travers la muqueuse intestinale et une libération lente de l'insuline. Des nanoparticules d'insuline préparées avec le même polymère (polyalkylcyanoacrylate) et administrées par voie orale n'ont montré aucune activité hypoglycémiante (COUVREUR P. et al, Acta Pharm. Technol., 26, (1980), 220-222).

De même, l'administration par voie orale de la cyclosporine est erratique et éminemment variable malgré la mise au point d'une forme pharmaceutique microémulsion (Neoral®, des laboratoires Novartis). La mise au point de nanoparticules de cyclosporine n'a actuellement pas permis d'augmenter la biodisponibilité orale de ce principe actif qui reste limitée à moins de 5% (FORD J., et al. Int. J. Pharm., 183, (1999), 3-6).

L'héparine est utilisée depuis une cinquantaine d'années dans la prévention et le traitement de la maladie thromboembolique, mais présente l'inconvénient d'être particulièrement hémorragique et de nécessiter une stricte surveillance biologique et clinique. En dehors d'anomalies fonctionnelles de l'hémostase qui entraînent un état d'hypercoagulabilité (déficit congénital ou acquis en anti-thrombine III, cofacteur II, protéines C et S), on note différents facteurs de risque de la maladie thromboembolique définie comme un ensemble de troubles de l'hémostase conduisant à la formation de caillot de fibrine ou de clous plaquettaires dans la lumière d'un vaisseau sanguin.

Ces facteurs de risque liés au patient sont :
- l'âge :la maladie atteint plus de 50% des sujets âgés de plus de 40 ans
- le sexe : fréquence de la maladie plus élevée chez la femme de moins de 40 ans, et surtout pendant la grossesse
- l'obésité
- la prise d'oestroprogestatifs
- le tabagisme
- l'hypertension artérielle
- le diabète
- l'hypercholestérolémie
- l'alitement qui favorise la stase
- les varices
- l'insuffisance cardiaque
- l'intervention chirurgicale : la fréquence de la maladie augmente en situation post-chirurgicale.

L'héparine est un mucopolysaccharide anionique sulfaté naturel constitué d'unités osidiques de D glucosamines et d'acides glucuroniques ou iduroniques, synthétisé par les mastocytes et extrait industriellement du poumon de boeuf ou de l'intestin de porc. Glucosamines et acides uroniques pouvant être substitués par des groupements sulfates ou acétyls déterminent ainsi une dizaine d'unités osidiques différentes. Ces différents motifs se répartissent de manière très cohérente, définissant trois régions intramoléculaires dont l'une d'entre elles est une structure pentasaccharidique, site d'action entre l'héparine et l'antithrombine III. En se liant à l'antithrombine III, l'héparine catalyse l'inactivation de plusieurs facteurs de coagulation, la thrombine et le facteur Xa en particulier. Il en résulte un allongement du temps de coagulation mesuré par le temps de céphaline activée. L'héparine est en fait une substance très hétérogène puisqu'elle comprend une mosaïque de molécules à chaîne saccharidique de poids moléculaire compris entre 2 500 et 40 000 daltons. Les chaînes polysaccharidiques de l'héparine naturelle peuvent être fractionnées par divers procédés (chromatographie, hydrolyse chimique et enzymatique), permettant d'obtenir des héparines de bas poids moléculaire (HBPM) dotées de propriétés originales qui les distinguent de l'héparine non fractionnée. Pour le praticien, les propriétés les plus importantes sont une demi-vie environ deux fois plus longue, un effet anticoagulant faible ou absent, une plus grande facilité d'administration par voie sous-cutanée, une meilleure tolérance locale, un faible pouvoir hémorragique et une pharmacocinétique plus longue.

L'administration de l'héparine se fait actuellement par voie parentérale, soit par voie intraveineuse ou sous-cutanée. Ce type d'administration est cependant contraignant et peut poser des problèmes d'observance pour les patients. De plus, une fois injectée par voie intraveineuse, l'héparine est rapidement éliminée de la circulation sanguine, et une dose importante doit être administrée à intervalles réguliers pour obtenir une action anticoagulante efficace, ce qui est souvent accompagné de saignements anormaux ou de complications telle qu'une thrombopénie.

Aussi, la possibilité d'administrer l'héparine par voie orale aurait en effet un impact important dans bon nombre de cas cliniques du domaine cardio-vasculaire.

Or, de par sa structure, l'héparine apparaît comme une molécule de haut poids moléculaire et comportant une forte densité de charges. Elle ne peut donc pas franchir aisément la barrière digestive après administration par voie orale.

Aussi, l'héparine administrée par voie orale n'est pas absorbée au niveau du tractus gastro-intestinal, et perd son activité anticoagulante en milieu acide (Morton et al., Int. J. Pharm., 9, (1981), 321-335, Doutremepuich et al., Seminars in Thrombosis and Hemostasis, 11 (3), (1985), 323-325). La stratégie utilisée a alors consisté à combler le manque d'absorption/activité anticoagulante par une augmentation importante de la dose administrée.

Ainsi, des études antérieures ont mis en évidence qu'après administration orale chez l'homme d'une grande quantité d'héparine en solution (40 000 UI, soit entre 10 et 17 fois la dose classiquement administrée par voie intraveineuse toutes les deux heures), seule une faible quantité est absorbée au niveau digestif et distribuée dans le sang. De plus, l'activité anticoagulante mesurée par le test du temps de céphaline activée (TCA) est très faible (Baughman et al., Circulation, 16, (1998), 1610-1615). De même chez l'homme, après administration par voie orale d'héparine de bas poids moléculaire (HBPM), aucune activité n'a été observée dans le plasma (Dryjski et al., Br. J. Clin. Pharmacol., 2, (1989), 188-192).

De nombreuses modifications chimiques de l'héparine et la préparation de différentes formulations ont été envisagées pour améliorer la biodisponibilité de l'héparine après administration par voie orale.

Dans un premier temps, des essais ont consisté à étudier des modifications de la structure de l'héparine (héparines de source différente, plus ou moins fragmentées, jusqu'à l'apparition des héparines de bas poids moléculaire).

Des solutions ont été préparées en complexant l'héparine avec des adjuvants tels que la lysine, la spermine ou la glycine, de façon à diminuer l'ionisation de l'héparine. Après administration orale, ces solutions ont montré une faible absorption de l'héparine (Tidball et al., Proc. Soc. Exp. Biol. Med. 111, (1962), 713-715).

Des solutions de sels acides de l'héparine ont également été préparées en associant à l'héparine des sels de sodium de l'acide éthylène diaminetétraacétique ou des sels biliaires (Morton et al., Int. J. Pharm., 9, (1981), 321-335).

Des émulsions huile dans eau (H/E) ou des solutions micellaires de sels de monooléine destinés à augmenter l'absorption de l'héparine ont également été envisagées (Taniguchi et al., Int. J. Pharm., 4, (1980), 219-228).

Des solutions de propylène glycol contenant de l'héparine et des composés dérivés de la N-acylation de l'acide aminé aromatique, l'acide 4-aminophénylbutyrique, ont démontré une amélioration de l'absorption gastrointestinale et de la biodisponibilité de l'héparine après administration orale à des rats et des singes (Leone-Bay et al., J. Controlled Red., 50, (1998), 41-49).

Bien que ces différentes solutions d'héparine (Tidball et al., Proc. Soc. Exp. Biol. Med. 111, (1962), 713-715, Morton et al., Int. J. Pharm., 9, (1981), 321-335, Taniguchi et al., Int. J. Pharm., 4, (1980), 219-228 et Leone-Bay et al., J. Controlled Red., 50, (1998), 41-49) aient permis, pour la plupart, d'améliorer l'absorption gastro-intestinale de l'héparine, l'effet anticoagulant observé est beaucoup plus faible et de durée inférieure à celui obtenu après administration par voie sous-cutanée pour des posologies très largement supérieures. De plus, le statut toxicopharmacologique des promoteurs d'absorption et des excipients utilisés compromet le succès de ces formulations.

Des gélules gastrorésistantes d'héparine ont été administrées à des lapins et une faible activité anti-Xa plasmatique (0,15 UI/ml) est observée entre la 2^{è} et la 4^{è} heure. Cependant, là encore, des doses très importantes d'héparine (15 000 UI anti-Xa/kg) sont administrées (Doutremepuich et al., Thérapie, 39, (1984), 147-152).

D'autres travaux ont consisté à chercher à optimiser l'absorption de l'héparine, et de ce fait, l'effet thérapeutique recherché. Cette nouvelle étape a été marquée par la fabrication de systèmes d'administration des médicaments, tels que les liposomes et les microparticules, qui ont permis d'envisager d'encapsuler l'héparine. Ces techniques d'encapsulation utilisées pour les enzymes, les médicaments et les hormones font que ces molécules restent plus longtemps dans la circulation sanguine que lorsqu'elles sont sous forme libre, du fait de leur relargage progressif à partir des systèmes polymériques et à la protection que ces derniers leur confèrent vis-à-vis de la dégradation enzymatique (Couvreur et al., Drug Del. Rev., 10, (1993), 141-162.

Des liposomes ont également été préparés et administrés à des chiens ; une absorption intestinale du principe actif a été observée, mais une faible activité biologique a été détectée alors que des doses toujours extrêmement importantes d'héparine étaient administrées (500 000 UI) (Ueno et a/., Chem. Pharm. Bull., 30 (6), (1982), 2245-2247).

Enfin, des microsphères composées d'acides aminés condensés thermiquement ont aussi été développées (Santiago et al., Proceed. Intern. Symp. Control. Rel. Mater., 19, (1992), 514-515 et Santiago et al., Proceed. Intern. Symp. Control. Rel. Bioact. Mater., 20, (1993), 300-301). Dans ce dernier cas la particule obtenue, dont la taille est comprise entre 0,5 et 10 µm, est appelée protéinoïde. Leur administration orale à des rats et des singes a permis de conclure à une absorption de l'héparine au niveau intestinal; ces résultats prometteurs se heurtent cependant à trois obstacles importants. Tout d'abord, l'activité biologique de l'héparine ne se manifeste qu'au maximum pendant 90 minutes. De plus, l'activité biologique a été obtenue chez le rat pour des doses plus de 10 fois supérieures à celles utilisées chez l'homme par voie parentérale. Enfin, les très nombreuses recherches actuelles d'immunisation par voie orale reposent sur le phénomène de capture, par les plaques de Peyer, de microparticules chargées en antigènes et dont la taille est comprise entre 1 et 10 µm. Dans ces conditions, les protéinoïdes utilisés pourraient induire un phénomène immunoallergique compromettant l'administration répétée de ces particules.

Les systèmes polymériques ont fait également l'objet de nombreuses études. Ainsi, Yang et al., (J. Control. Rel., 60, (1999), 269-277) ont préparé des microparticules d'héparine à base uniquement de polymères d'acide lactique et d'acide glycolique (PLGA) dans le but d'inhiber la prolifération des cellules musculaires lisses des vaisseaux sanguins lors d'une étude in vitro (Yang et al., J. Control. Rel., 60, (1999), 269-277). Les microparticules, fabriquées par une technique de nébulisation *(spray-drying),* ont une taille très faible (entre 3 et 9 µm). La libération de l'héparine in vitro est très lente (entre 10 et 40 jours), ce qui est incompatible avec une administration orale pour laquelle le temps de transit est de l'ordre de 24 à 48 h.

WO 96 28143 A décrit des compositions pharmaceutiques sous forme de microparticules contenant des polypeptides et notamment de l'insuline comme principe actif. Ces particules sont formées à partir d'un mélange de polymères biodégradables (copolymère de l'acide lactique et de l'acide glycolique) et de polymères polycationiques (polyamino acides : polyarginine et polyhistidine ou chitosan).

WO 92 11844 A décrit des microparticules contenant un peptide ou une protéine comme principe actif et un mélange de polymères formé d'un polymère biodégradable (e.g. polymères de l'acide lactique, copolymères de l'acide lactique et de l'acide glycolique, polyamides, polyanhydrides) et d'un polymère polycationique (polyarginine, polylysine ou chitosan).

Toutefois, tous ces essais utilisent des doses très importantes d'héparine par rapport à ce qui est classiquement utilisé chez l'homme en thérapeutique. Aussi existe-t-il encore un besoin de mettre à la disposition des patients, un système d'administration qui augmente l'absorption des principes actifs, notamment de l'héparine, après administration orale et permet d'administrer lesdits-principes actifs à des concentrations moins élevées diminuant ainsi les effets secondaires néfastes.

Or, les Inventeurs ont montré de manière surprenante, et Inattendue au regard de la perméatlon de grosses molécules à travers la barrière gastrointestinale, que des vecteurs particulaires comprenant une matrice polymérique à base d'un mélange de polymère biodégradable non entérique et d'un polymère polycationique non entérique permettent d'administrer par voie orale des quantités de principes actifs, notamment d'héparine proches de celles classiquement utilisées par voie parentérale.

Aussi, la présente Invention a pour objet des vecteurs particulaires destinés à améliorer l'absorption orale de principes actifs, formés d'une matrice polymérique comprenant au moins un polymère biodégradable associé à au moins un polymère polycationique.

Au sens de la présente invention, les polymères biodégradables et les polymères polycationiques peuvent être gastro-résistants (entériques) ou non.

Dans un autre mode particulier de réalisation de l'invention le polymère biodégradable et le polymère polycationique sont présents en quantité équivalente.

Avantageusement, le polymère biodégradable non entérique est choisi dans le groupe constitué par les polyesters, notamment les polymères de l'acide lactique, les copolymères de l'acide lactique et de l'acide glycolique (PLGA), la poly-s-caprolactone (PCL), les polyanhydrides, les poly(amides), les poly(uréthanes), les poly(carbonates), les poly(acétals), les poly(orthoesters), et les polymères naturels (collagène, polysaccharides)...).

Avantageusement, le polymère polycationique est choisi dans le groupe constitué par les dérivés de la cellulose, les copolymères d'esters des acides acryliques et méthacryliques commercialisés par la firme Rhôm GmBh sous le nom d'Eudragit® et plus particulièrement les polyesters de l'acide méthacrylique avec une faible proportion de chlorure de triméthylammonioéthyle méthacrylate (Eudragit® RS) ou une proportion plus importante de chlorure de triméthylammonioéthyle méthacrylate (Eudragit® RL), le chitosane et ses dérivés et la polylysine.

Dans un mode particulièrement avantageux de l'invention, le polymère biodégradable est soit PCL, soit PLGA le poids moléculaire desdits polymères étant compris entre 2 000 et 100 000.

Dans un mode particulier de réalisation selon l'invention, les vecteurs particulaires se présentent soit sous forme de nanoparticules dont le diamètre est compris entre 50 et 1000 nm, de préférence entre 200 et 400 nm, soit sous forme de microparticules dont le diamètre est compris entre 1 et 1000 µm, de préférence entre 50 et 200 µm.

Selon l'invention, la matrice polymérique peut comprendre en outre une ou plusieurs substances choisies dans le groupe comprenant les polymères entériques, les agents tensioactifs et les substances hydrosolubles ou liposolubles.

Dans un mode particulier de réalisation de l'invention, le principe actif est choisi dans le groupe constitué par l'héparine et les produits apparentés, les héparines de bas poids moléculaire (HBPM) et les produits apparentés, les peptides et les protéines, notamment l'insuline, la cyclosporine, les oligonucléotides antisens, l'ADN et l'hormone de croissance.

Dans un autre mode particulier de réalisation de l'invention, le vecteur particulaire permet l'administration par voie orale de l'héparine standard à une dose comprise entre 2 000 UI et 20 000 UI/jour et l'HBPM à une dose comprise entre 600 UI et 4 200 UI/jour.

La présente invention a également pour objet une composition pharmaceutique qui contient un vecteur particulaire tel que décrit précédemment en association avec tout excipient pharmaceutiquement acceptable.

Les compositions peuvent être utilisées une ou plusieurs fois par jour, sous toutes les formes adaptées à l'administration orale, notamment sous forme de gélules, de comprimés, de granulés, de sachets ou de lyophilisat.

Les compositions selon l'invention permettent d'administrer les principes actifs à des doses équivalentes à environ 1 à 10 fois la dose utilisée par voie parentérale. De tels vecteurs permettent de supprimer les inconvénients de la voie parentérale (stérilisation du médicament, douleur au point d'injection, angoisse du patient, risque d'infection, nombre de points d'injection limité). Ils évitent en outre, comme c'est souvent le cas par voie orale, d'administrer des doses très importantes de principes actifs puisque les principes actifs sont utilisés à une dose équivalente à celle utilisée classiquement par voie intraveineuse ou très légèrement supérieure, au maximum 10 fois, de préférence 1 à 3 fois la dite dose.

D'autre part, l'emploi de polymères considérés comme biocompatibles (biodégradables et/ou non biodégradables) est une garantie de l'absence de toxicité desdites particules.

De façon inattendue, les vecteurs particulaires selon l'invention permettent en outre d'obtenir une action plus prolongée que l'administration d'une dose similaire en solution administrée par voie intraveineuse, alors qu' il est connu que les doses administrées par voie orale doivent souvent être largement supérieures aux doses administrées par voie intraveineuse pour pouvoir exercer leur activité, en raison des pertes en principe actif occasionnées par leur séjour dans le tractus gastro-intestinal (pH acide de l'estomac, enzymes, sécrétions diverses, premier passage hépatique ...).

Conformément à l'invention, les vecteurs particulaires peuvent être préparés par toute méthode connues de l'homme du métier. On peut citer à titre d'exemple la méthode de préparation par émulsification et évaporation de solvant telle que décrite par Alex et al., (J. Microencapsulation, 7 (3), (1990), 347-355). D'autres méthodes peuvent également être envisagées notamment la nébulisation *(spray-drying),* l'enrobage et l'extrusion.

Les exemples et les figures qui suivent illustrent l'invention sans toutefois la limiter.

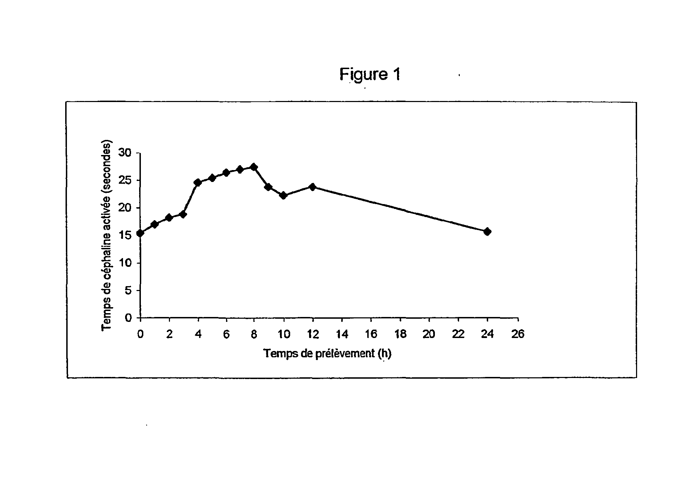

La figure 1 illustre l'activité biologique de l'héparine déterminée par le temps de céphaline activée après administration orale de microparticules d'héparine préparées à partir du mélange de polymères Eudragit® RS/PLGA dans des proportions (1/1) selon le mode opératoire de l'exemple 1 et administrées selon le mode opératoire de l'exemple 9.

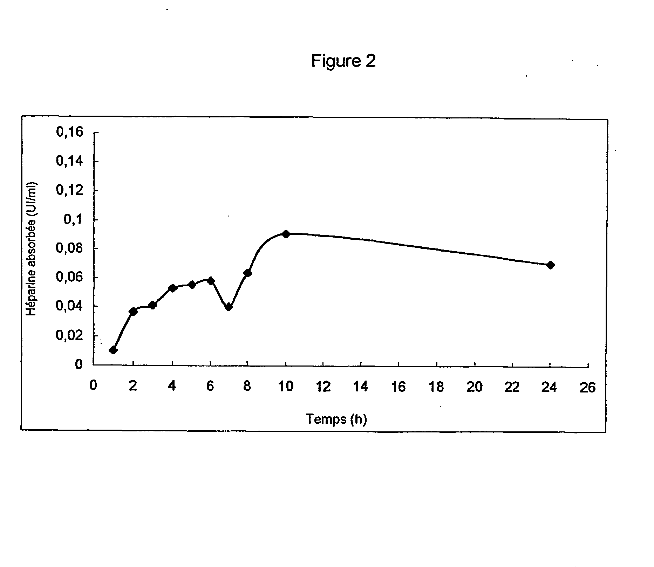

La figure 2 illustre l'héparinémie après administration orale de nanoparticules d'héparine préparées à partir du mélange de polymères Eudragit® RL/PCL dans de proportions (1/1) selon le mode opératoire de l'exemple 1 et administrées selon le mode opératoire de l'exemple 9.

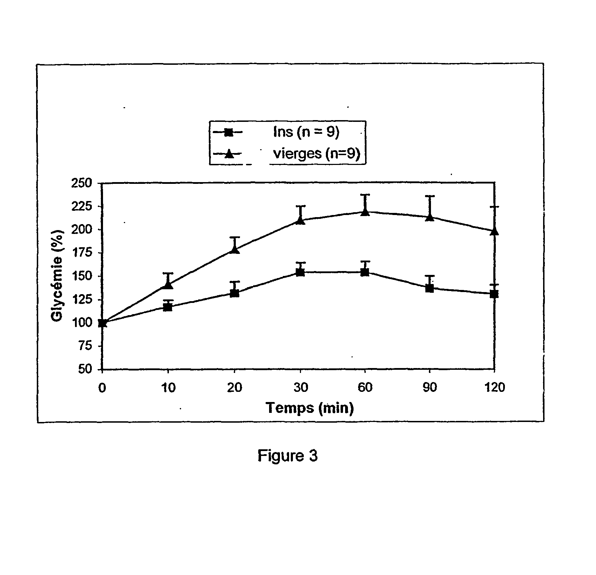

La figure 3 illustre la cinétique de la glycémie induite par une administration de 2 g de glucose administré par voie orale, 4 heures après l'administration orale de nanoparticules d'insuline préparées selon le mode opératoire de l'exemple 10 (vierges = rats non traités; ins = rats traités par nonoparticules selon l'invention).

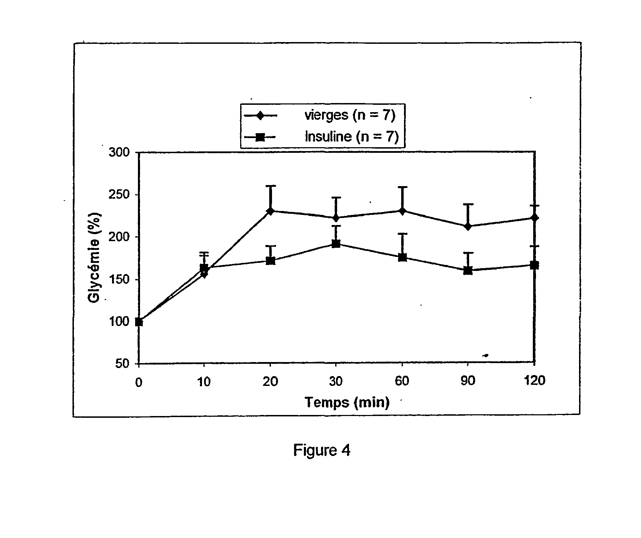

La figure 4 illustre la cinétique de la glycémie induite par une administration de 2 g de glucose administré par voie orale, 8 heures après l'administration orale de nanoparticules d'insuline préparées selon le mode opératoire de l'exemple 10 (vierges = rats non traités; ins = rats traités par nonoparticules selon l'invention).

### Exemple 1: Préparation de vecteurs particulaires contenant de l'héparine (microparticules)

La solution d'héparine standard ou de bas poids moléculaire (1 ml, 5 000 UI) est émulsionnée sous agitation magnétique pendant 3 min (500 rpm) dans une solution de dichlorométhane (10 ml) contenant le polymère ou le mélange de polymères (250 mg). Cette première émulsion (eau/huile) est ensuite versée dans un volume d'eau (1500 ml) contenant un agent tensioactif, l'alcool polyvinylique (0,1% degré d'hydrolyse 88 %), permettant d'obtenir sous agitation mécanique (2000 tours/min) une seconde émulsion eau/huile/eau. Après 2 heures d'agitation, la précipitation des gouttelettes dispersées est obtenue après évaporation du solvant. Les microparticules polymères ainsi obtenues sont ensuite isolées par filtration. Les particules ont une taille moyenne de 150 µm.

### Exemple 2: Préparation de vecteurs particulaires contenant de l'héparine et de la gélatine A (microparticules)

On procède selon l'exemple 1 avec addition de gélatine A (0,5%) dans la solution d'héparine.

### Exemple 3: Préparation de vecteurs particulaires contenant de l'héparine et du chlorure de sodium (microparticules)

On procède selon l'exemple 1 avec addition de NaCl (0,2%) dans la solution d'héparine.

### Exemple 4: Préparation de vecteurs particulaires contenant de l'héparine (nanoparticules)

La solution d'héparine standard ou de bas poids moléculaire (1 ml, 5 000 UI) est émulsionnée à l'aide d'une sonde à ultra-sons pendant 3 min dans une solution de dichlorométhane (10 ml) contenant le polymère ou le mélange de polymères (250 mg). Cette première émulsion (eau/huile) est ensuite versée dans un volume d'eau (200 ml) contenant un agent tensioactif, l'alcool polyvinylique (0,1%), permettant d'obtenir par homogénéisation sous pression (homogénéisateur à filière) une seconde émulsion eau/huile/eau. Après 3 minutes de cisaillement, l'agitation est arrêtée et le solvant est évaporé de la suspension colloïdale dans un évaporateur sous pression réduite, entraînant la formation de nanoparticules polymères en suspension dans l'eau. La suspension des nanoparticules est lavée 3 trois par centrifugation (25000 g). Cette suspension peut être utilisée telle quelle ou lyophilisée. Les particules ont une taille moyenne de 250 nm.

### Exemple 5: Préparation de vecteurs particulaires contenant de l'héparine et de la gélatine A (nanoparticules)

On procède selon l'exemple 4 avec addition de gélatine A (0,5%) dans la solution d'héparine.

### Exemple 6: Préparation de vecteurs particulaires contenant de l'héparine et du chlorure de sodium (nanoparticules)

On procède selon l'exemple 4 avec addition de NaCl (0,2%) dans la solution d'héparine.

### Exemple 7: Caractérisation physico-chimiques des particules

Des microparticules et des nanoparticules sont préparées selon les modes opératoires des exemples 1 à 6 et contiennent au total 0,25 g de polymères ou de mélange de polymères.

Les caractéristiques pour les microparticules sont rassemblées dans le tableau 1 comme étant la moyenne de 3 essais (moyenne ± écart-type).

Les caractéristiques pour les nanoparticules sont rassemblées dans le tableau 2 comme étant la moyenne de 4 essais (moyenne ± écart-type).

La charge en principe actif (exprimée en pourcentage et en UI d'héparine/gramme de polymère) dans et/ou sur lesdites particules est déterminée par une méthode colorimétrique validée avec une solution d'Azure II dans le cas de l'héparine standard non fractionnée et par néphélométrie dans le cas des HBPM (héparine de bas poids moléculaire).

Le diamètre des microparticules et des nanoparticules a été obtenu par les moyens classiques de diffraction/diffusion de la lumière connues de l'homme du métier.

Le potentiel de surface des nanoparticules a été déterminé par électrophorèse laser.

Les résultats montrent que la technique de fabrication est très reproductible.

Pour les microparticules et les nanoparticules préparées uniquement avec un polymère biodégradable, les taux d'incorporation de l'héparine sont faibles, ce qui exigerait, même si on supposait une absorption, des quantités trop importantes et incompatibles de particules (de l'ordre de plusieurs grammes en une seule administration).

En revanche, les microparticules selon l'invention présentent un taux d'incorporation suffisant pour permettre d'administrer des quantités compatibles de particules.

**Tableau 1**

| Type de polymère Quantité = 0,25 g | Taux d'incorporation | | Taille moyenne (µm) |
|---|---|---|---|
| | % | UI/g de polymère | |
| Eudragit®RS PO^{a} | 49,29 ± 4,03 | 9952 ± 798,1 | 96,23 |
| Eudragit® RL PO^{a} | 79,89 ± 3,45 | 15956 ± 687,6 | 79,76 |
| PCL^{a} | 23,55 ± 3,51 | 4566 ± 699,7 | 128,33 |
| PLGA^{a} | 26,81 ± 3,76 | 1929 ± 160,9 | 125,16 |
| PCL/PLGA (1/1)^{a} | 17,52 ± 4,69 | 3506 ± 928,8 | 82,04 |
| RS/RL (1/1)^{a} | 66,59 ± 1,54 | 13307 ± 303,1 | 88,07 |
| RS/RL/PLGA (1/1/2) ^{b} | 45,13 ± 2,85 | 8984 ± 568,7 | 71,07 |
| RS/PLGA (1/1) ^{b} | 52,48 ± 4,17 | 10517 ± 908,2 | 86,98 |
| RL/PLGA (1/1) ^{b} | 63,79 ± 3,95 | 12752 ± 785,3 | 128,51 |
| RS/RL/PCL (1/1/2) ^{b} | 40,39 ± 2,42 | 8126 ± 508,7 | 104,38 |
| RS/PCL (1/1) ^{b} | 36,27 ± 3,72 | 7277 ± 722,0 | 129,36 |
| RL/PCL (1/1) ^{b} | 45,16 ± 2,13 | 9032 ± 466,7 | 103,45 |
| RS/gélatine A (5%) ^{a} | 66,63 ± 4,06 | 13323 ± 811,2 | 123,64 |
| RS/NaCI (2%) ^{a} | 16,40 ± 2,41 | 3186 ± 394,6 | 84,99 |
| RS/gélatine B (5%)^{a} | 46,31 ± 1,54 | 9260 ± 299,9 | 80,40 |
| PCL/gélatine A (5%) ^{a} | 57,96 ± 4,73 | 11577 ± 928,4 | 201,04 |
| PCL/Naa (2%) ^{a} | 17,09 ± 1,90 | 3416 ± 377,5 | 90,89 |
| PCL/gélatine B (5%) ^{a} | 24,08 ± 2,72 | 4826 ± 536,7 | 124,96 |
| PLGA/gélatine A (5%) ^{a} | 58,71 ± 3,94 | 11735 ± 786,8 | 282,24 |
| PLGA/NaCl (2%) ^{a} | 22,59 ± 3,34 | 4517 ± 666,1 | 107,97 |
| PLGA/gélatine B (5%) ^{a} | 37,51 ± 2,41 | 7505 ± 491,6 | 130,43 |

| | | | |
|---|---|---|---|
| ^{a} exemples comparatifs ^{b} exemples selon invention | | | |

**Tableau 2**

| Type de polymère Quantité = 0.25 g | Héparine encapsulée en UI/g de polymère (%) | Potentiel de surface (mV) | Taille (nm) (polydispersité) |
|---|---|---|---|
| Eudragit® RL^{a} | 19477 490,2 (97,38 ±2,45) | -38,8 ± 2,4 | 265,7 ± 8,22 (0,102) |
| Eudragit® RS^{a} | 11825 ± 139,6 (59,13 ± 0,71) | -22,4 ± 0,45 | 268,5 ± 15,83 (0,110) |
| PCL ^{a} | 1673 ± 208,8 (8,36 ± 1,06) | -1,6 ± 0,22 | 285,3 ± 9,92 (0,064) |
| PLGA ^{a} | 2792 ± 800,5 (13,97 ± 4,01) | -4,5 ± 0,07 | 266,5 ± 4,00 (0,058) |
| RS/PLGA (1/1) ^{b} | 7101 ± 430,9 (35,53 ± 2,15) | -17,3 ± 1,35 | 273,4 ± 7,37 (0,083) |
| RL/PLGA (1/1) ^{b} | 9752 ± 720,8 (48,78 ± 3,60) | -37,2 ± 3,30 | 268,9 ± 8,13 (0,10) |
| RS/RL/PLGA^{b} (1/1/2) | 7498 ± 138,4 (37,55 ± 0,69) | -30,7 ± 2,02 | 275,4 ± 3,41 (0,074) |
| RS/PCL (1/1) ^{b} | 5657 ± 324,0 (28,30 ± 1,61) | -20,0 ± 0,67 | 285,9 ± 5,88 (0,07) |
| RL/PCL (1/1) ^{b} | 10663 ± 320,6 (53,36 ± 1,64) | -33,6 ± 1,93 | 303,6 ± 3,49 (0,086) |
| RS/RL/PCL (1/1/2) ^{b} | 7645 ± 588,4 (38,25 ± 2,94) | -29,9 ± 0,39 | 295,0 ± 4,34 (0,088) |
| RS/RL ^{a} | 14287 ± 448,3 (71,44 ± 2,21) | -35,7 ± 1,94 | 269,6 ± 7,07 (0,088) |
| PCL/PLGA ^{a} | 853 ± 158,4 (4,26 ± 0,79) | 2,5 ± 0,38 | 264,9 ± 0,61 (0,061) |
| RS/gélatine A ^{a} | 11891 ± 741,2 (59,43 ± 3,42) | -18,6 ± 1,21 | 279,9 ± 3,12 (0,099) |
| PCL/gélatine A ^{a} | 7414 ± 870,4 (37,04 ± 4,35) | -2,58 ± 0,23 | 284,4 ± 3,90 (0,091) |
| PLGA/gélatine A ^{a} | 8533 ± 701,7 (41,31 ± 3,51) | -4,4 ± 0,31 | 274,4 ± 3,22 (0,073) |

| | | | |
|---|---|---|---|
| ^{a} exemples comparatifs ^{b} exemples selon invention | | | |

### Exemple 8: Quantité d'héparine libérée in vitro

L'activité biologique de l'héparine encapsulée puis libérée des particules préparées selon les exemples 1 à 6 a été déterminée par une méthode chronométrique (TCA, temps de céphaline activée ; kit C.K. Prest®, Diagnostica Stago) et une méthode chromogénique (activité anti-Xa ; kit Stachrom® Heparin, Diagnostica Stago) selon les instructions du fabricant.

Les résultats obtenus montrent que les valeurs obtenues pour la quantité d'héparine libérée sont identiques par les deux méthodes, ce qui confirme que l'héparine a conservé son activité biologique après encapsulation.

### Exemple 9: Etude in vivo après administration orale à des lapins

Les résultats sont donnés dans les figures 1 et 2.

Des gélules contenant les particules polymères d'héparine préparées selon les exemples 1 à 6 à partir de 250 mg de polymères ou de mélanges de polymères sont administrées en dose unique, 2 000 UI pour l'héparine standard, 600 UI pour l'HBPM à des lapins à jeun depuis 12 heures. Des prélèvements sanguins (500 µl) sont effectués au temps T₀ et à des temps réguliers au niveau de la veine externe de l'oreille. Après centrifugation de chaque échantillon sanguin à 7 000 g pendant 8 min, le temps de céphaline activée ou l'activité anti-Xa sont déterminés comme indiqué dans l'exemple 8.

De façon inattendue, en administration unique par voie orale, et avec une concentration de 20 à 250 fois plus faible en principe actif que celles utilisées dans l'état antérieur de la technique [2 000 UI d'héparine alors que les autres travaux publiés font état de doses allant de 40 000, 60 000 à 90 000 toutes les 8 heures pendant 5 jours (Baugham, Proceed, Intern. Symp. Control. Rel. Bioact. Mater., 26, (1999), 4) jusqu'à 500 000 UI (Ueno et al., Chem. Pharm., 30 (6), (1982), 2245-2247)], les particules d'héparine préparées selon la présente invention augmentent de façon significative et prolongée le temps de coagulation.

En revanche, après administration orale de microparticules ou nanoparticules préparées uniquement à partir de polymères biodégradables (PLGA, PCL...),aucune absorption de l'héparine standard n'a été constatée. Après administration orale de microparticules ou nanoparticules préparées uniquement à partir de polymères non biodégradables (Eudragit® RL, Eudragit® RS) aucune absorption de l'héparine standard n'a été observée.

Ainsi les vecteurs particulaires d'héparine de la présente invention permettent d'administrer par voie orale des doses pratiquement équivalentes à celles administrées actuellement chez l'homme par voie intraveineuse et sous-cutanée, tout en assurant une efficacité prolongée du principe actif.

### Exemple 10: Préparation de vecteurs particulaires contenant de l'insuline (nanoparticules)

La solution d'insuline est émulsionnée à l'aide d'une sonde à ultrasons pendant 30 secondes dans une solution de dichlorométhane contenant le mélange de polymères (250 mg).

Cette première émulsion eau/huile est ensuite versée dans un volume d'eau (40 ml) contenant un tensioactif, l'alcool polyvinylique (0,1%) et émulsionnée à l'aide d'une sonde à ultrasons pendant 1 minute, obtenant ainsi une seconde émulsion eau/huile/eau.

Le solvant organique est ensuite évaporé à l'aide d'un évaporateur sous pression réduite, entraînant la formation des nanoparticules. La suspension colloïdale est centrifugée pendant 30 minutes (42 000 g), le surnageant est éliminé et les nanoparticules sont remises en suspension dans l'eau et utilisées telles quelles. Les particules ont une taille moyenne de 350 nm.

### Exemple 11: Etude in vivo après administration orale à des rats diabétiques de nanoparticules d'insuline

Les résultats sont représentés par les figures 3 e 4.

La suspension de nanoparticules d'insuline préparée selon l'exemple 10 est administrée par voie orale en dose unique (100 Ul/kg) à des rats rendus diabétiques par administration de streptozacine et à jeun depuis 12 heures. Un test d'hyperglycémie provoqué (2g de glucose administrés par voie orale) est effectué 4 et 8 heures après administration orale des nanoparticules. Des prélèvements sanguins sont effctués au temps T₀ et à des temps réguliers au niveau de la veine de la queue. La glycémie et l'insulinémie sont déterminées pour chaque échantillon sanguin.

De façon inattendue, après administration unique par voie orale, les particules d'insuline préparées selon la présente invention diminuent de façon significative la glycémie.

Parallèlement, on observe une augmentation de l'insulinémie.

## Revendications

1. Vecteurs particulaires destinés à améliorer l'absorption orale de principes actifs, lesdits vecteurs étant formés d'une matrice polymérique comprenant au moins un polymère biodégradable associé à au moins un polymère polycationique, **caractérisés en ce que** ledit polymère biodégradable et ledit polymère polycationique sont présent en quantité équivalente.

2. Vecteurs particulaires selon la revendication 1, **caractérisés en ce que** le polymère biodégradable non entérique est choisi dans le groupe constitué par les polyesters, notamment les polymères de l'acide lactique, les copolymères de l'acide lactique et de l'acide glycolique (PLGA), la poly-ε-caprolactone (PCL) et les polyanhydrides, les poly(amides), les poly(uréthanes), les poly(carbonates), les poly(acétals), les poly(orthoesters) et les polymères naturels.

3. Vecteurs particulaires selon la revendication 2, **caractérisés en ce que** le polymère biodégradable non entérique est PCL ou PLGA, le poids moléculaire desdits polymères étant compris entre 2,000 et 100,000.

4. Vecteurs particulaires selon l'une quelconque des revendications 1 à 3, **caractérisés en ce que** le polymère polycationique est choisi dans le groupe constitué par les dérivés de la cellulose, les copolymères d'esters des acides acryliques et méthacryliques commercialisés par la firme Rhöm GmbH sous le nom d'Eudragit® et plus particulièrement les polyesters de l'acide méthacrylique avec une faible proportion de chlorure de triméthylammonioéthyle méthacrylate (Eudragit®RS) ou une proportion plus importante de chlorure de triméthyulammonioéthyle méthacrylate (Eudragit®RL), le chitosane et ses dérivés et la polylysine.

5. Vecteurs particulaires selon l'une quelconque des revendications 1 à 4, **caractérisés en ce qu'**ils se présentent sous forme de nanoparticules ou de microparticules.

6. Vecteurs particulaires selon la revendication 5 **caractérisés en ce qu'**ils se présentent sous forme de nanoparticules dont le diamètre est compris entre 50 et 1000 nm.

7. Vecteurs particulaires selon la revendication 6, **caractérisés en ce qu'**ils se présentent sous forme de nanoparticules dont le diamètre est compris entre 200 et 400 nm:

8. Vecteurs particulaires selon la revendication 5, **caractérisés en ce qu'**ils se présentent sous forme de microparticules dont le diamètre est compris entre 1 et 1000 µm.

9. Vecteurs particulaires selon la revendication 8, **caractérisés en ce qu'**ils se présentent sous forme de microparticules dont le diamètre est compris entre 50 et 200 µm.

10. Vecteurs particulaires selon l'une quelconque des revendications 1 à 9, **caractérisés en ce qu'**ils comprennent en outre une ou plusieurs substances choisies dans le groupe comprenant les polymères entériques et les substances hydrosolubles ou liposolubles.

11. Vecteurs particulaires selon l'une quelconque des revendications 1 à 10, **caractérisés en ce que** le principe actif est choisi parmi l'héparine et les produits apparentés, les héparines de bas poids moléculaire et les produits apparentés, les peptides et les protéines.

12. Vecteurs particulaires selon la revendication Il, **caractérisés en ce que** le principe actif est l'héparine.

13. Composition pharmaceutique **caractérisé en ce qu'**elle contient au moins un vecteur particulaire selon l'une quelconque des revendications 1 à 12, en association avec tout excipient pharmaceutiquement acceptable.

14. Composition pharmaceutique selon la revendication 13, **caractérisé en ce qu'**elle est préparée sous forme adaptée à l'administration orale.

15. Composition pharmaceutique selon la revendication 14, **caractérisé en ce qu'**elle est préparée sous forme de gélules, de comprimés, de granulés, de sachets ou de lyophilisats.

16. Utilisation de vecteurs particulaires selon l'une quelconque des revendications 1 à 12 pour la préparation d'un médicament.

17. Utilisation de vecteurs particulaires selon la revendication 16 pour la préparation d'un médicament destiné à l'administration par voie orale des protéines, des peptides, de l'héparine et les produits apparentés, et des héparines de bas poids moléculaire et les produits apparentés

## Claims

1. Particulate vectors for improving oral absorption of active principles, said vectors being made up of a polymeric matrix comprising at least one biodegradable polymer associated with at least one polycationic polymer, **characterised in that** said biodegradable polymer and said polycationic polymer are present in an equivalent amount.

2. Particulate vectors according to Claim 1, **characterized in that** the nonenteric biodegradable polymer is chose from the group being constituted of polyesters, in particular lactic acid polymers, copolymers of lactic acid and of glycolic acid (PLGA), poly-ε-caprolactone (PCL) and polyanhydrides, poly(amide)s, poly(urethyane)s, poly (carbonate) s, poly(acetal)s, poly(orthoester)s and natural polymers,

3. Particulate vectors according to Claim 2, **characterized in that** the nonenteric biodegradable polymer is PCL or PLGA, the molecular weight of said polymers being between 2000 and 100 000.

4. Particulate vectors according to any one of Claims 1 to 3, **characterized in that** the polycationic polymer is chosen from the group being constituted of cellulose derivatives, copolymers of acrylic and methacrylic acid esters sold by the company Rhöm GmbH under the name Eudragit^{®}, and more particularly polyesters of methacrylic acid with a low proportion of trimethylammonicethyl methacrylate chloride (Eudragit^{®}RS) or a higher proportion of trimethylammonicethyl methacrylate chloride (Eudragit^{®}RL), chitosan and its derivatives, and polylysine.

5. Particulate vectors according to any one of Claims 1 to 4, **characterised in that** they are in the form of nanoparticles or of microparticles.

6. Particulate vectors according to claim 5, **characterized in that** they are in the form of nanoparticles, the diameter of which is between 50 and 1000 nm.

7. Particulate vectors according to Claim 6, **characterized in that** they are in the form of nanoparticles, the diameter of which is between 200 and 400 nm.

8. Particulate vectors according to Claim 5, **characterized in that** they are in the form of microparticles, the diameter of which is between 1 and 1000 µm.

9. Particulate vectors according to Claim 8, **characterized in that** they are in the form of microparticles, the diameter of which is between 50 and 200 µm.

10. Particulate vectors according to any one of Claims 1 to 9, **characterized in that** they also comprise one or more substances chosen from the group comprising enteric polymers and watersoluble or liposoluble substances.

11. Particulate vectors according to any one of Claims 1 to 10, **characterised in that** the active principle is chosen from heparin and related products, low-molecular-weight heparins and related products, peptides and proteins.

12. Particulate vectors according to Claim 11, **characterized in that** the active principle is heparin.

13. pharmaceutical composition, **characterized in that** it contains at least one particulate vector according to any one of Claims 1 to 12, in combination with any pharmaceutically acceptable excipient.

14. Pharmaceutical composition according to Claim 13, **characterized in that** it is prepared in a form suitable for oral administration.

15. Pharmaceutical composition according to Claim 14, **characterized in that** it is prepared in the form of gel capsules, tablets, granules, sachets or freeze-dried materials.

16. Use of particulate vectors according to any one of Claims 1 to 12, for the preparation of a medicament.

17. Use of particulate vectors according to Claim 16, for the preparation of a medicament for use in oral administration of proteins, of peptides, of heparin and related products, and of low-molecular-weight heparins and related products.

## Patentansprüche

1. Teilchenförmige Träger zur Verbesserung der oralen Absorption von Wirkstoffen, wobei die Träger in Form einer polymeren Matrix vorliegen, die mindestens ein biologisch abbaubares Polymer umfasst, das mit mindestens einem polykationischen Polymer assoziiert ist, **dadurch gekennzeichnet, dass** das biologisch abbaubare Polymer und das polykationische Polymer in äquivalenten Mengen vorliegen.

2. Teilchenförmige Träger nach Anspruch 1, **dadurch gekennzeichnet, dass** das nicht-enterische biologisch abbaubare Polymer ausgewählt ist aus der Gruppe bestehend aus Polyestern, insbesondere Milchsäurepolymeren, Milchsäure- und Glycolsäure-Copolymeren (PLGA), Poly-ε-caprolacton (PCL) und Polyanhydriden, Poly(amiden), Poly(urethanen), Poly(carbonaten), Poly(acetalen), Poly(orthoestern) und natürlichen Polymeren.

3. Teilchenförmige Träger nach Anspruch 2, **dadurch gekennzeichnet, dass** das nicht-enterische biologisch abbaubare Polymer PCL oder PLGA ist, wobei die Molmasse der Polymere zwischen 2000 und 100 000 liegt.

4. Teilchenförmige Träger nach einem der Ansprüche 1 bis 3, **dadurch gekennzeichnet, dass** das polykationische Polymer ausgewählt ist aus der Gruppe bestehend aus Cellulosederivaten, Copolymeren von Estern von Acrylsäure und Methacrylsäure, die von der Firma Rhöm GmbH unter der Bezeichnung Eudragit^{®} vertrieben werden, und insbesondere Polyestern von Methacrylsäure mit einem kleinen Anteil an Trimethylammoniumethylmethacrylat-Chlorid (Eudragit^{®} RS) oder einem größeren Anteil an Trimethylammoniumethylmethacrylat-Chlorid (Eudragit^{®} RL), Chitosan und dessen Derivaten und Polylysin.

5. Teilchenförmige Träger nach einem der Ansprüche 1 bis 4, **dadurch gekennzeichnet, dass** sie in Form von Nanopartikeln oder Mikropartikeln vorliegen.

6. Teilchenförmige Träger nach Anspruch 5, **dadurch gekennzeichnet, dass** sie in Form von Nanopartikeln vorliegen, deren Durchmesser zwischen 50 und 1000 nm liegt.

7. Teilchenförmige Träger nach Anspruch 6, **dadurch gekennzeichnet, dass** sie in Form von Nanopartikeln vorliegen, deren Durchmesser zwischen 200 und 400 nm liegt.

8. Teilchenförmige Träger nach Anspruch 5, **dadurch gekennzeichnet, dass** sie in Form von Mikropartikeln vorliegen, deren Durchmesser zwischen 1 und 1000 µm liegt.

9. Teilchenförmige Träger nach Anspruch 8, **dadurch gekennzeichnet, dass** sie in Form von Mikropartikeln vorliegen, deren Durchmesser zwischen 50 und 200 µm liegt.

10. Teilchenförmige Träger nach einem der Ansprüche 1 bis 9, **dadurch gekennzeichnet, dass** sie darüber hinaus ein oder mehrere Substanzen umfassen, die ausgewählt sind aus der Gruppe bestehend aus enterischen Polymeren und wasserlöslichen oder fettlöslichen Substanzen.

11. Teilchenförmige Träger nach einem der Ansprüche 1 bis 10, **dadurch gekennzeichnet, dass** der Wirkstoff aus Heparin und verwandten Produkten, Heparinen mit einer niedrigen Molmasse und verwandten Produkten, Peptiden und Proteinen ausgewählt ist.

12. Teilchenförmige Träger nach Anspruch 11, **dadurch gekennzeichnet, dass** der Wirkstoff Heparin ist.

13. Pharmazeutische Zusammensetzung, **dadurch gekennzeichnet, dass** sie mindestens einen teilchenförmigen Träger nach einem der Ansprüche 1 bis 12 in Verbindung mit einem beliebigen pharmazeutisch annehmbaren Trägermittel enthält.

14. Pharmazeutische Zusammensetzung nach Anspruch 13, **dadurch gekennzeichnet, dass** sie in einer Form hergestellt ist, die für die orale Verabreichung angepasst ist.

15. Pharmazeutische Zusammensetzung nach Anspruch 14, **dadurch gekennzeichnet, dass** sie in Form von Kapseln, Tabletten, Säckchen oder Lyophilisaten hergestellt ist

16. Verwendung von teilchenförmigen Trägern nach einem der Ansprüche 1 bis 12 zur Herstellung eines Medikaments.

17. Verwendung von teilchenförmigen Trägern nach Anspruch 16 zur Herstellung eines Medikaments, das zur oralen Verabreichung von Proteinen, Peptiden, Heparin und verwandten Produkten und Heparinen mit niedriger Molmasse und verwandten Produkten vorgesehen ist.
